# EUROPÄISCHE PATENTSCHRIFT

(11) **EP 3 847 456 B1**
(45) Veröffentlichungstag und Bekanntmachung des Hinweises auf die Patenterteilung: **15.05.2024**
(21) Anmeldenummer: 19762977.7
(22) Anmeldetag: 04.09.2019
(51) Int. Cl.: G01N 33/28, G01N 1/22

(54) **ANALYSE EINES IN EINEM ISOLIERMEDIUM EINES HOCHSPANNUNGSGERÄTS GELÖSTEN GASES**
ANALYSIS OF A GAS DISSOLVED IN AN INSULATING MEDIUM OF A HIGH-VOLTAGE DEVICE
ANALYSE D'UN GAZ DISSOUS DANS UN MILIEU ISOLANT D'UN APPAREIL À HAUTE TENSION

(30) Priorität: 05.09.2018 DE 102018121647
(43) Veröffentlichungstag der Anmeldung: 14.07.2021
(73) Patentinhaber: Maschinenfabrik Reinhausen GmbH, 93059 Regensburg (DE)
(72) Erfinder: KLINKERT, Marcell, 55118 Mainz (DE); RUOFF, Patrick, 76131 Karlsruhe (DE)
(86) Internationale Anmeldenummer: PCT/EP2019/073589
(87) Internationale Veröffentlichungsnummer: WO 2020/049049

(56) Entgegenhaltungen:
- CN-A- 103 926 299
- KR-B1- 101 761 838
- US-A- 6 037 592
- US-B1- 6 421 588
- BELANGER G ET AL: "Laboratory Testing of a Sensor for Hydrogen Dissolved in Transformer Oil", IEEE TRANSACTIONS ON ELECTRICAL INSULATION, IEEE, US, Bd. EI-1, Nr. 2, April 1981 (1981-04), Seiten 144-148, XP011162494, ISSN: 0018-9367
- TSUKIOKA H ET AL: "New Apparatus for Detecting H2, CO, and CH4 Dissolved in Transformer Oil", IEEE TRANSACTIONS ON ELECTRICAL INSULATION, IEEE, US, Bd. EI-1, Nr. 4, August 1983 (1983-08), Seiten 409-419, XP011162710, ISSN: 0018-9367

## Beschreibung

Die Erfindung betrifft ein Verfahren und eine Analysevorrichtung zur Analyse eines in einem Isoliermedium eines Hochspannungsgeräts gelösten Gases.

Die Konzentration bestimmter gelöster Gase in einem Isoliermedium, zum Beispiel einem Isolieröl, eines Hochspannungsgeräts, beispielsweise eines Transformators oder eines Stufenschalters, kann als Indikator für Fehlfunktionen oder bevorstehende Fehlfunktionen des Hochspannungsgeräts dienen. In diesem Zusammenhang sind beispielsweise Wasserstoff und Kohlenmonoxid relevant und können auf elektrische Fehler beziehungsweise Fehler in Isolationsmaterialien hinweisen. Eine Analyse der Gaskonzentration im Isoliermedium, insbesondere eine dauerhafte Online-Überwachung, ermöglicht die frühzeitige Erkennung von möglicherweise problematischen Entwicklungen.

Bestehende Verfahren beziehungsweise Vorrichtungen zur Analyse gelöster Gase im Isoliermedium eines Hochspannungsgeräts berücksichtigen beispielsweise materialbedingte Produkt- oder Produktionsschwankungen nicht und erreichen daher nur eingeschränkte Genauigkeit.

Das Dokument US 6 037 592 A beschäftigt sich mit einem Sensorsystem zum Messen der Konzentration von in einer Flüssigkeit gelösten Gas in einem Transformator. Dabei verwendet das System eine passive Gasextraktionstechnik und einen Infrarot-basierten Sensor zum Nachweis vorhandener Gase in einer Flüssigkeit. Auch Druck- und Temperaturwerte werden für die Berechnung der Konzentrationen der Fehlergasbestandteile im extrahierten Gas berücksichtigt.

Dokument CN 103 926 299 A1 beschreibt eine online-Überwachung von ölgefüllten elektrischen Geräten mit einem Gassensor zur gleichzeitigen Erfassung eines Feuchtigkeitsgehalts, der Öltemperatur und eines Fehlergasgehalts in Isolieröl.

Dokument US 6 421 588 B1 beschreibt ein Selbstdiagnosesystem zur Bestimmung eines Wartungszustands von Schmiermitteln in ölverbrauchenden Maschinen, die unter intermittierender Last arbeiten. Mittels Headspace-Technologie werden Dämpfe über dem Gasraum des Motors entnommen und von einer Sensoranordnung analysiert.

Die Publikation BELANGER G ET AL: "Laboratory Testing of a Sensor for Hydrogen Dissolved in Transformer Oil", IEEE TRANSACTIONS ON ELECTRICAL INSULATION, IEEE, US, Bd. EI-1, Nr. 2, April 1981 (1981-04), Seiten 144-148, XP011162494, ISSN: 0018-9367 beschreibt einen Sensor zur Echtzeitmessung der Wasserstoffkonzentration in dem Isolieröl eines Transformators mit verbesserten Verhalten.

Das Dokument KR 101 761 838 B1 beschreibt einen Gassensor zur Echtzeitmessung einer Wasserstoffgaskonzentration in einem Isolieröl eines Transformators, wobei der Sensor im Isolieröl platziert ist.

Die Publikation TSUKIOKA H ET AL:"New Apparatus for Detecting H2, CO, and CH4 Dissolved in Transformer Oil", IEEE TRANSACTIONS ON ELECTRICAL INSULATION, IEEE, US, Bd. EI-1, Nr. 4, August 1983 (1983-08), Seiten 409-419, XP011162710, ISSN: 0018-9367 beschreibt eine Methode zur Detektion von verschiedenen Gasen in einem Transformatoröl, wobei die Gase mittels einer Membran extrahiert und mithilfe eines katalytischen Gassensors und einem Gaschromatographen detektiert werden.

Es ist daher eine Aufgabe der vorliegenden Erfindung ein verbessertes Konzept für die Analyse eines in einem Isoliermedium eines Hochspannungsgeräts gelösten Gases anzugeben, welches zu einer verbesserten Analysegenauigkeit führt.

Diese Aufgabe wird durch den jeweiligen Gegenstand der unabhängigen Ansprüche gelöst. Weitere Ausführungsformen sind Gegenstand der abhängigen Ansprüche.

Das verbesserte Konzept beruht auf der Idee, ein mehrstufiges Verfahren zur Auswertung eines Sensorsignals umzusetzen und in den einzelnen Stufen unterschiedliche Einflüsse der Vorrichtung und der Umgebung auf ein Sensorsignal zu berücksichtigen. Die einzelnen Stufen sind dabei in sich voneinander unabhängig. Dadurch können gerätespezifische Verhaltensweisen differenziert betrachtet und kompensiert werden.

Gemäß dem verbesserten Konzept wird ein Verfahren zur Analyse eines in einem Isoliermedium eines Hochspannungsgeräts gelösten Gases angegeben. Dabei wird das gelöste Gas mittels einer Analysevorrichtung, insbesondere einer Separationseinheit einer Analysevorrichtung, in eine Gasphase überführt, um ein Analysegasgemisch zu erhalten. Das Analysegasgemisch wird zu einem Analysesensor der Analysevorrichtung transportiert. Ein Sensorsignal wird als Ausgangssignal des Analysesensors abhängig von einer Zusammensetzung des Analysegasgemisches, insbesondere von einer Konzentration des in die Gasphase überführten Gases in dem Analysegasgemisch, erzeugt. Basierend auf dem Sensorsignal und wenigstens einer Störgröße und einer weiteren Störgröße wird eine erste Zwischengröße erzeugt. Eine zweite Zwischengröße wird basierend auf der ersten Zwischengröße und wenigstens einer spezifischen Größe der Analysevorrichtung bestimmt. Ein Wert für eine Konzentration des gelösten Gases in dem Isoliermedium wird basierend auf der zweiten Zwischengröße bestimmt.

Das Analysegasgemisch kann außer dem in die Gasphase überführten Gas ein Trägergas enthalten, beispielsweise Luft. Das Trägergas kann beispielsweise mittels einer Pumpvorrichtung in die Analysevorrichtung gebracht werden.

Eine Störgröße spiegelt dabei beispielsweise äußere Einflüsse oder Zustände wider, welche insbesondere nicht von Produktschwankungen beispielsweise des Analysesensors oder anderer Komponenten der Analysevorrichtung herrühren.

Dagegen spiegelt die wenigstens eine spezifische Größe der Analysevorrichtung Eigenschaften der Analysevorrichtung selbst wider, beispielsweise aufgrund von Produkt- oder Produktionsschwankungen, insbesondere materialbedingte Schwankungen. Insbesondere ist die wenigstens eine spezifische Größe der Analysevorrichtung unabhängig von äußeren Einflüssen oder Zuständen.

Die separate Berücksichtigung der wenigstens einen Störgröße, der weiteren Störgröße und der wenigstens einer spezifischen Größe der Analysevorrichtung erlaubt eine differenzierte Berücksichtigung der Eigenschaften der Analysevorrichtung selbst, beispielsweise bei der Modellierung der Gaskonzentration in dem Isoliermedium.

Gemäß zumindest einer Ausführungsform handelt es sich bei dem Isoliermedium um eine Isolierflüssigkeit, insbesondere Isolieröl, beispielsweise ein Transformatoröl, oder eine alternative Isolierflüssigkeit, wie zum Beispiel einen synthetischen Ester.

Gemäß zumindest einer Ausführungsform handelt es sich bei dem Hochspannungsgerät um einen Transformator, insbesondere einen Leistungstransformator, eine Drossel oder einen Stufenschalter, beispielsweise einen Laststufenschalter, zur Umschaltung zwischen verschiedenen Wicklungsanzapfungen eines Transformators oder einer Drossel.

Gemäß zumindest einer Ausführungsform beinhaltet das gelöste Gas Wasserstoff oder Kohlenmonoxid.

Gemäß zumindest einer Ausführungsform beeinflusst die wenigstens eine spezifische Größe der Analysevorrichtung das Überführen des gelösten Gases in die Gasphase.

Insbesondere hängt dann eine Konzentration des Gases in der Gasphase im Inneren der Analysevorrichtung von einer Konzentration des gelösten Gases in dem Isoliermedium sowie von der wenigstens einen spezifischen Größe der Analysevorrichtung ab.

Gemäß zumindest einer Ausführungsform beeinflusst die wenigstens eine spezifische Größe der Analysevorrichtung ein Verhalten des Analysesensors, insbesondere die Erzeugung des Ausgangssignals. Insbesondere hängt dann das Sensorsignal von einer Konzentration des gelösten Gases in dem Isoliermedium sowie von der wenigstens einen spezifischen Größe der Analysevorrichtung ab.

Gemäß zumindest einer Ausführungsform wird das Überführen des gelösten Gases in die Gasphase mittels einer semipermeablen Membran der Analysevorrichtung, insbesondere der Separationseinheit, durchgeführt. Die wenigstens eine spezifische Größe der Analysevorrichtung umfasst eine Eigenschaft der Membran, insbesondere eine Porosität der Membran.

Gemäß zumindest einer Ausführungsform umfasst die wenigstens eine spezifische Größe der Analysevorrichtung eine Eigenschaft des Analysesensors, insbesondere eine Empfindlichkeit des Analysesensors, eine Ansprechzeit des Analysesensors oder Reaktionszeit des Analysesensors.

Gemäß zumindest einer Ausführungsform beeinflusst die wenigstens eine Störgröße ein Verhalten des Analysesensors. Insbesondere hängt das Ausgangssignal von einem Eingangssignal des Analysesensors sowie von der wenigstens einen Störgröße ab.

Erfindungsgemäß umfasst die Störgröße eine Temperatur und einen Feuchtegehalt einer Umgebung des Analysesensors.

Gemäß zumindest einer Ausführungsform umfasst die Störgröße auch einen Druck einer Umgebung des Analysesensors. Bei der Umgebung kann es sich insbesondere um das Innere einer Detektionseinheit der Analysevorrichtung handeln, in welcher der Analysesensor angeordnet ist.

Gemäß zumindest einer Ausführungsform umfasst das Verfahren das Spülen der Analysevorrichtung mit einem Spülgas sowie das Bestimmen eines Spülergebnisses, insbesondere einer Qualität oder eines Erfolgsgrades der Spülung. Dann kann die wenigstens eine Störgröße eine Größe zur Bestimmung des Spülergebnisses umfassen.

Gemäß zumindest einer Ausführungsform wird das Bestimmen des Wertes für die Konzentration zusätzlich basierend auf wenigstens einer weiteren Störgröße durchgeführt.

Gemäß zumindest einer Ausführungsform beeinflusst die wenigstens eine weitere Störgröße das Überführen des gelösten Gases in die Gasphase. Insbesondere hängt dann eine Konzentration des Gases in der Gasphase im Inneren der Analysevorrichtung von einer Konzentration des gelösten Gases in dem Isoliermedium sowie von der wenigstens einen weiteren Störgröße ab.

Gemäß zumindest einer Ausführungsform umfasst die wenigstens eine weitere Störgröße, die bei der Bestimmung der ersten Zwischengröße verwendet wird, eine Temperatur des Isoliermediums, einen Feuchtegehalt des Isoliermediums und/oder einen Druck des Isoliermediums, erfindungsgemäß jedoch zumindest eine Strömungsgeschwindigkeit des Isoliermediums.

Gemäß zumindest einer Ausführungsform wird das Bestimmen der zweiten Zwischengröße zusätzlich basierend auf einer spezifischen Größe des Isoliermediums durchgeführt. Hierbei handelt es sich um einen Materialparameter des Isoliermediums. Dieser kann zwar möglicherweise von Umgebungsbedingungen abhängen, stellt jedoch selbst keine solche dar.

Gemäß zumindest einer Ausführungsform beeinflusst die spezifische Größe des Isoliermediums das Überführen des gelösten Gases in die Gasphase.

Gemäß zumindest einer Ausführungsform umfasst die spezifische Größe des Isoliermediums eine Löslichkeit des gelösten Gases in dem Isoliermedium.

Gemäß der Erfindung wird die erste Zwischengröße basierend auf einem ersten Regressionsmodell bestimmt. Die zweite Zwischengröße wird basierend auf einem zweiten Regressionsmodell bestimmt. Der Wert für die Konzentration des gelösten Gases wird basierend auf einem dritten Modell, insbesondere Regressionsmodell, bestimmt. Dabei sind die drei Modelle unabhängig voneinander, was eine dedizierte Berücksichtigung und Kompensation der Störgrößen, weiteren Störgrößen und/oder spezifischen Größen der Analysevorrichtung erlaubt und damit zu einer verbesserten Genauigkeit führt.

Gemäß dem verbesserten Konzept wird auch eine Analysevorrichtung zur Analyse eines in einem Isoliermedium eines Hochspannungsgeräts gelösten Gases angegeben. Insbesondere ist die Analysevorrichtung zur Durchführung eines Verfahrens nach dem verbesserten Konzept geeignet.

Die Analysevorrichtung umfasst eine Separationseinheit, einen Analysesensor, eine Pumpvorrichtung und eine Auswerteeinheit. Die Separationseinheit ist dazu eingerichtet, das gelöste Gas in eine Gasphase zu überführen um ein Analysegasgemisch zu erhalten. Die Pumpvorrichtung ist dazu eingerichtet, das Analysegasgemisch von der Separationseinheit zu dem Analysesensor zu transportieren. Der Analysesensor ist dazu eingerichtet, ein Sensorsignal Ausgangssignal abhängig von einer Zusammensetzung des Analysegasgemisches zu erzeugen.

Die Auswerteeinheit ist dazu eingerichtet, eine erste Zwischengröße basierend auf dem Sensorsignal und wenigstens einer Störgröße und einer weiteren Störgröße und eine zweite Zwischengröße basierend auf der ersten Zwischengröße und wenigstens einer spezifischen Größe der Analysevorrichtung zu bestimmen. Außerdem ist die Auswerteeinheit dazu eingerichtet einen Wert für eine Konzentration des gelösten Gases in dem Isoliermedium basierend auf der zweiten Zwischengröße zu bestimmen.

Gemäß zumindest einer Ausführungsform der Analysevorrichtung umfasst die Separationseinheit eine semipermeable Membran, durch welche das gelöste Gas von dem Hochspannungsgerät in die Analysevorrichtung gelangen kann.

Gemäß zumindest einer Ausführungsform umfasst der Analysesensor einen Metall-Oxid-Halbleiter-Feldeffekttransistor, MOSFET.

Gemäß zumindest einer Ausführungsform hängt das Sensorsignal von einem Widerstand, insbesondere einem Source-Drain Widerstand, des MOSFET ab. Insbesondere ist der Widerstand umso kleiner, je größer die Konzentration des Gases in dem Analysegasgemisch ist.

Gemäß zumindest einer Ausführungsform hängt das Sensorsignal von einem Widerstand des MOSFET nach einer Sättigungsdauer ab. Dabei entspricht die Sättigungsdauer beispielsweise einem Zeitraum zwischen Ende der Spülung und einem Zeitpunkt, an dem sich der Wert des Widerstands nicht mehr signifikant ändert.

Der MOSFET oder ein weiterer MOSFET kann beispielsweise auch dazu eingesetzt werden, das Ergebnis der Spülung zu bestimmen oder zu bewerten.

Gemäß zumindest einer Ausführungsform umfasst die Auswerteeinheit eine Prozessoreinheit und eine Messeinrichtung zur Bestimmung einer Temperatur der Prozessoreinheit. Die Auswerteeinheit ist dazu eingerichtet, eine Temperatur in einer Umgebung des Analysesensors, beispielsweise innerhalb der Detektionseinheit, basierend auf der Temperatur der Prozessoreinheit zu bestimmen. Dazu kann sich die Prozessoreinheit beispielsweise entsprechend nahe an dem Analysesensor befinden.

In diesem Fall kann vorteilhaft auf einen zusätzlichen Temperatursensor in der Detektionseinheit verzichtet werden um die Temperatur der Umgebung des Analysesensors zu bestimmen.

Gemäß zumindest einer Ausführungsform ist die Auswerteeinheit dazu eingerichtet, die Temperatur in der Umgebung des Analysesensors basierend auf der Temperatur der Prozessoreinheit und auf weiteren Einflussgrößen, insbesondere weiterer Wärmequellen, beispielsweise Relais oder Isoliermedium, zu bestimmen

Weitere Ausgestaltungsformen und Implementierungen der Analysevorrichtung gemäß dem verbesserten Konzept ergeben sich unmittelbar aus den verschiedenen Ausgestaltungsformen des Verfahrens nach dem verbesserten Konzept und umgekehrt.

Im Folgenden wird die Erfindung anhand beispielhafter Ausführungsformen unter Bezug auf die Zeichnungen im Detail erklärt.

Es zeigen
- Figur 1: einen schematischen Aufbau einer beispielhaften Ausführungsform einer Analysevorrichtung gemäß dem verbesserten Konzept; und
- Figur 2: ein Ablaufdiagramm einer beispielhaften Ausgestaltung eines Verfahrens nach dem verbesserten Konzept.

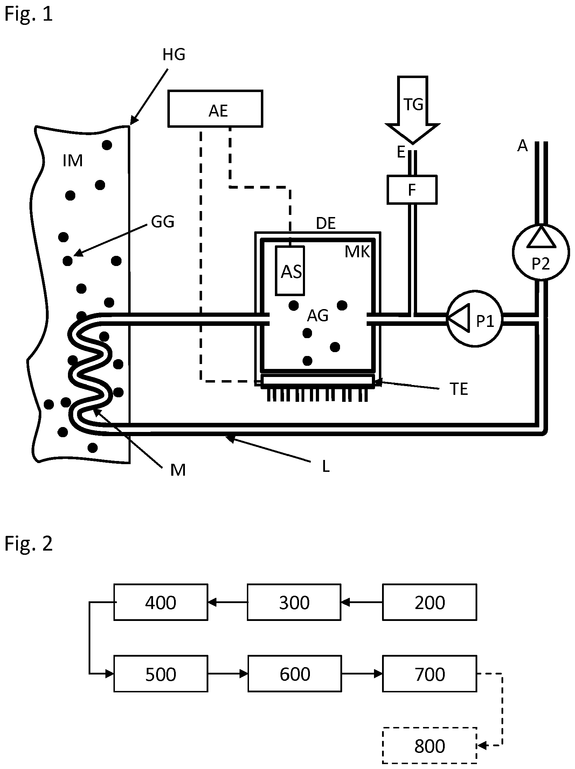

In Figur 1 ist ein schematischer Aufbau einer beispielhaften Ausführungsform einer Analysevorrichtung gemäß dem verbesserten Konzept gezeigt. Figur 2 zeigt ein zugehöriges Ablaufdiagramm einer beispielhaften Ausgestaltung eines Verfahrens nach dem verbesserten Konzept.

Figur 1 zeigt ein Hochspannungsgerät HG, etwa einen Leistungstransformator oder einen Laststufenschalter, mit einem Tank oder Gefäß, welches zumindest teilweise mit einem Isoliermedium IM, beispielsweise Transformatoröl, gefüllt ist. In dem Isoliermedium können verschieden Gase gelöst vorliegen. Im Folgenden beschränkt sich die Diskussion auf ein bestimmtes gelöstes Gas GG, beispielsweise Wasserstoff oder Kohlenmonoxid. Die Analysevorrichtung kann jedoch in verschiedenen Ausführungsformen mehrere verschiedene Gase analysieren, insbesondere Wasserstoff und Kohlenmonoxid. Die folgenden Ausführungen gelten daher für verschiedene Gase analog.

Die Analysevorrichtung umfasst eine Separationseinheit, welche im direkten Kontakt mit dem Isoliermedium steht. Dazu kann die Separationseinheit wenigstens teilweise durch entsprechende Durchführungen des Hochspannungsgerät ins Innere desselben geführt werden. Im gezeigten Beispiel umfasst die Separationseinheit eine semipermeable Membran M.

Die Analysevorrichtung umfasst eine Detektionseinheit DE, welche beispielsweise eine Messkammer MK aufweist. Im Inneren der Detektionseinheit DE, insbesondere der Messkammer MK ist ein Analysesensor AS angeordnet.

Außerdem umfasst die Analysevorrichtung einen Einlass E für ein Trägergas TG, einen Gasauslass A, sowie optional einen Filter F zur Filterung des durch den Einlass E einströmenden Trägergases TG.

Die Analysevorrichtung umfasst eine Pumpvorrichtung, welche beispielsweise eine erste Pumpe P1 und eine zweite Pumpe P2 umfasst.

Die Analysevorrichtung umfasst ein Leitungssystem L, welches die Separationseinheit, gegebenenfalls die Membran M, die Detektionseinheit DE, insbesondere die Messkammer MK, den Einlass E, den Auslass A und die Pumpvorrichtung miteinander verbindet.

Optional umfasst die Detektionseinrichtung DE eine Temperiereinrichtung TE, beispielsweise ein Peltier-Element, zur Temperierung des Inneren der Detektionseinrichtung DE, insbesondere der Messkammer MK und der Umgebung des Analysesensors AS.

Optional umfasst die Detektionseinrichtung DE einen Drucksensor (nicht gezeigt) zur Bestimmung des Inneren der Detektionseinrichtung DE, insbesondere der Messkammer MK und der Umgebung des Analysesensors AS.

Die Analysevorrichtung umfasst eine Auswerteeinheit AE, welche mit dem Analysesensor AS gekoppelt ist.

Gegebenenfalls kann die Auswerteeinheit AE auch zur Steuerung der Temperiereinrichtung TE mit dieser gekoppelt sein, falls letztere vorhanden ist. Alternativ kann eine separate Steuereinheit (nicht gezeigt) zur Steuerung der Temperiereinrichtung TE vorgesehen sein. In diesem Fall kann die Steuereinheit auch zur Steuerung der Pumpvorrichtung dienen.

Im Betrieb der Analysevorrichtung kann im Schritt 200 der Figur 2 das gelöste Gas GG durch die Membran M ins Innere der Analysevorrichtung, insbesondere in das Leitungssystem L, gelangen, wo es dann in der Gasphase vorliegt. Das flüssige Isoliermedium IM kann dagegen die Membran M nicht passieren. Zudem kann die Pumpeinrichtung das Trägergas TG in das Leitungssystem transportieren, so dass es mit dem Gas, welches durch die Membran M gelangt ist, ein Analysegasgemisch AG bildet.

Dieser Prozess der Diffusion kann durch ein Konzentrationsgefälle oder ein chemisches Potenzial getrieben sein. Es bildet sich beispielsweise ein dynamisches Gleichgewicht an der Membran M bezüglich der Gaskonzentration aus. Dieses Gleichgewicht ist beispielsweise abhängig von der Temperatur des Isoliermediums IM, der Konzentration des gelösten Gases GG im Isoliermedium IM und/oder der Löslichkeit des Gases im Isoliermedium IM.

Mit steigender Temperatur steigt die Prozessgeschwindigkeit. Auch kann das Niveau des Gleichgewichtszustandes dadurch beeinflusst werden. Über die Gaskonzentration im Isoliermedium IM wird der Konzentrationsgradient verändert. Dabei kann die Gaskonzentration im Isoliermedium IM lokal variabel sein. So kann sich bei nicht vorhandener Konvektion des Isoliermediums IM eine Verarmungszone an gelöstem Gas GG um die Membran M ausbilden. Eine Konvektion unterstützt eine Oberflächenerneuerung der Gase an der Membran M. Die Löslichkeit des Gases GG im Isoliermedium IM ist abhängig von der Gesamtheit an gelösten Stoffen im Isoliermedium M. So hat beispielsweise ein Anteil an gelöstem Wasser im Isoliermedium M einen Einfluss darauf, wie gut Wasserstoff gelöst werden kann.

Alternativ zur Membran M kann die Separationseinheit andere Vorrichtungen umfassen, welche eine Trennung des gelösten Gases GG von dem Isoliermedium IM bewirken können. Beispielsweise kann die Trennung mittels der Vakuum-Methode oder der Headspace-Methode durchgeführt werden. Die beiden letztgenannten Methoden sind dem Fachmann bekannt.

Das Analysegasgemisch AG wird im Schritt 300 mittels der Pumpvorrichtung ins Innere der Detektionseinheit DE und damit zu dem Analysesensor AS transportiert.

Im Schritt 400 wird mittels des Analysesensors AS ein Sensorsignal erzeugt, welches beispielsweise einem Sättigungswert eines Widerstands des Analysesensors AS entspricht. Das Sensorsignal ist dabei abhängig von einer Konzentration des zu analysierenden Gases in dem Analysegasgemisch AG und damit von der Konzentration des gelösten Gases GG in dem Isoliermedium.

Optional kann vor der Erzeugung des Sensorsignals das Leitungssystem L und die Detektionseinheit DE, insbesondere die Messkammer MK, mittels der Pumpvorrichtung mit Trägergas TG gespült werden. Dadurch kann erzielt werden, dass das Analysegasgemisch AG zunächst im Wesentlichen aus dem Trägergas TG besteht und nach der Spülung die Konzentration des zu analysierenden Gases zunimmt bis hin zu einem Gleichgewichtszustand. Dadurch kann das Sensorsignal normalisiert werden, indem es beispielsweise dem Unterschied zwischen dem Ausgangssignal des Analysesensors AS direkt nach der Spülung und nach Erreichen des Gleichgewichts beziehungsweise der Sättigung entspricht.

Das Sensorsignal wird von der Auswerteeinheit AE erfasst. Im Schritt 500 bestimmt die Auswerteeinheit AE eine erste Zwischengröße basierend auf dem Sensorsignal und wenigstens einer Störgröße und einer weiteren Störgröße, welche beispielsweise ein Verhalten des Analysesensors AS beeinflusst. Die Störgröße beinhaltet erfindungsgemäß eine Temperatur und einen Feuchtegehalt zum Beispiel Z im Inneren der Detektionseinheit DE oder der Messkammer MK.

Zusätzlich kann sie auch einen Druck beinhalten. Die Störgröße kann beispielsweise im Schritt 400 von der Auswerteeinheit AE erfasst werden. Dazu kann die Analysevorrichtung weitere Sensoren (nicht gezeigt) zur Bestimmung der Störgröße umfassen, welche mit der Auswerteeinheit AE gekoppelt sind. Zur Bestimmung der ersten Zwischengröße wird dabei ein erstes Regressionsmodell eingesetzt. Die weitere Störgröße umfasst erfindungsgemäß zumindest eine Strömumgsgeschwindigkeit des Isomiermediums IM.

Die Auswerteeinheit AE bestimmt dann in Schritt 600 eine zweite Zwischengröße basierend auf der ersten Zwischengröße und wenigstens einer spezifischen Größe der Analysevorrichtung, welche das Überführen des gelösten Gases GG in die Gasphase und/oder ein Verhalten des Analysesensors AS beeinflusst. Im ersten Fall kann die wenigstens eine spezifische Größe der Analysevorrichtung eine Eigenschaft der Separationseinheit, beispielsweise der Membran M, beispielsweise eine Porosität der Membran M, umfassen. Im zweiten Fall kann die wenigstens eine spezifische Größe der Analysevorrichtung eine Eigenschaft des Analysesensors AS, beispielsweise eine Empfindlichkeit des Analysesensors AS, umfassen. Die wenigstens eine spezifische Größe der Analysevorrichtung kann während des Verfahrens nach dem verbesserten Konzept bestimmt werden oder vor der Inbetriebnahme der Analysevorrichtung. So können beispielsweise material- oder prozessabhängige Produktschwankungen differenziert berücksichtigt und kompensiert werden. Zur Bestimmung der zweiten Zwischengröße wird ein zweites Regressionsmodell eingesetzt.

Im Schritt 700 bestimmt die Auswerteeinheit AE basierend auf der zweiten Zwischengröße und optional zusätzlich basierend auf wenigstens einer weiteren Störgröße einen Wert für eine Konzentration des gelösten Gases GG in dem Isoliermedium IM. Die wenigstens eine weitere Störgröße beeinflusst dabei das Überführen des gelösten Gases GG in die Gasphase. Die wenigstens eine weitere Störgröße kann beispielsweise eine Temperatur, einen Feuchtegehalt und/oder eine Strömungsgeschwindigkeit des Isoliermediums IM beinhalten. Sie kann beispielsweise im Schritt 200 von der Auswerteeinheit AE erfasst werden. Dazu kann die Analysevorrichtung oder das Hochspannungsgerät HG weitere Sensoren (nicht gezeigt) zur Bestimmung der wenigstens einen weiteren Störgröße umfassen, welche mit der Auswerteeinheit AE gekoppelt sind. Zur Bestimmung des Wertes für die Konzentration kann dabei beispielsweise ein drittes Regressionsmodell eingesetzt werden.

In einem optionalen Schritt 800 kann die Auswerteeinheit AE basierend auf dem Wert für die Konzentration eine Handlungsanweisung oder eine Information erzeugen, beispielsweise ein Warnsignal oder ähnliches.

Mit einem Verfahren beziehungsweise einer Analysevorrichtung gemäß dem verbesserten Konzept kann in der beschriebenen Weise die Analyse eines in einem Isoliermedium eines Hochspannungsgeräts gelösten Gases mit einer verbesserten Analysegenauigkeit durchgeführt werden.

Die Trennung der Analyse in unabhängige Stufen erlaubt es außerdem, ein linearisiertes Modell für die Kalibrierung der Analysevorrichtung in der Produktion zu verwenden. Das Modell kann beispielsweise über eine einfache Zwei-Punkt-Kalibrierung gerätespezifisch eingestellt werden. Im Feld kann eine Ein-Punkt-Justierung im Feld angewendet werden (zum Beispiel besitzt degeneriertes Isolieröl eine andere Gaslöslichkeit als frisches Isolieröl).

### BEZUGSZEICHEN

- HG: Hochspannungsgerät
- IM: Isoliermedium
- GG: gelöstes Gas
- M: semipermeable Membran
- L: Leitungssystem
- AE: Auswerteeinheit
- DE: Detektionseinheit
- MK: Messkammer
- AS: Analysesensor
- AG: Analysegasgemisch
- TE: Temperiereinrichtung
- P1, P2: Pumpen
- E: Einlass
- A: Auslass
- TG: Trägergas
- F: Filter

## Patentansprüche

1. Verfahren zur Analyse eines in einem Isoliermedium (IM) eines Hochspannungsgeräts (HG) gelösten Gases (GG), wobei das Verfahren umfasst
- Überführen des gelösten Gases (GG) mittels einer Analysevorrichtung in eine Gasphase um ein Analysegasgemisch (AG) zu erhalten;
- Transportieren des Analysegasgemisches (AG) zu einem Analysesensor (AS) der Analysevorrichtung;
- Erzeugen eines Sensorsignals als Ausgangssignal des Analysesensors (AS) abhängig von einer Zusammensetzung des Analysegasgemisches (AG), wobei die Zusammensetzung des Analysegasgemisches (AG) einer Konzentration eines in eine Gasphase überführten Gases in dem Analysegasgemisch (AG) entspricht;
- Bestimmen einer ersten Zwischengröße basierend auf dem Sensorsignal, welches von der Konzentration des in die Gasphase überführten Gases in dem Analysegasgemisch (AG) abhängig ist und wenigstens einer Störgröße und einer weiteren Störgröße, anhand eines ersten Regressionsmodells, wobei die erste Zwischengröße eine von der Störgröße und weiteren Störgröße beeinflusste Konzentration des in die Gasphase überführten Gases ist und die wenigstens eine Störgröße eine Temperatur und ein Feuchtegehalt einer Umgebung des Analysesensors (AS) und die weitere Störgröße eine Strömungsgeschwindigkeit des Isoliermediums (IM) umfasst;
- Bestimmen einer zweiten Zwischengröße basierend auf der ersten Zwischengröße und wenigstens einer spezifischen Größe der Analysevorrichtung anhand eines zweiten Regressionsmodells, wobei die wenigstens eine spezifische Größe der Analysevorrichtung eine Eigenschaft des Analysesensors (AS) oder der Analysevorrichtung umfasst; und
- Bestimmen eines Wertes für eine Konzentration des gelösten Gases (GG) in dem Isoliermedium (IM) basierend auf der zweiten Zwischengröße.

2. Verfahren nach Anspruch 1, wobei die wenigstens eine spezifische Größe der Analysevorrichtung das Überführen des gelösten Gases (GG) in die Gasphase oder ein Verhalten des Analysesensors (AS) beeinflusst.

3. Verfahren nach einem der Ansprüche 1 oder 2, wobei das Überführen des gelösten Gases (GG) in die Gasphase mittels einer semipermeablen Membran (M) der Analysevorrichtung durchgeführt wird und die wenigstens eine spezifische Größe der Analysevorrichtung eine Eigenschaft der Membran (M) umfasst, insbesondere eine Porosität der Membran (M).

4. Verfahren nach einem der Ansprüche 1 bis 3, wobei die Eigenschaft des Analysesensors (AS) eine Empfindlichkeit, eine Ansprechzeit oder Reaktionszeit des Analysesensors (AS) ist .

5. Verfahren nach einem der Ansprüche 1 bis 4, wobei die wenigstens eine Störgröße ein Verhalten des Analysesensors (AS) beeinflusst.

6. Verfahren nach einem der Ansprüche 1 bis 5, wobei das Bestimmen der zweiten Zwischengröße zusätzlich basierend auf einer spezifischen Größe des Isoliermediums (IM) durchgeführt wird.

7. Verfahren nach Anspruch 6, wobei die spezifische Größe des Isoliermediums (IM) das Überführen des gelösten Gases (GG) in die Gasphase beeinflusst.

8. Verfahren nach einem der Ansprüche 6 oder 7, wobei die spezifische Größe des Isoliermediums (IM) eine Löslichkeit des gelösten Gases (GG) in dem Isoliermedium (IM) umfasst.

9. Analysevorrichtung zur Analyse eines in einem Isoliermedium eines Hochspannungsgeräts (HG) gelösten Gases (GG), die Analysevorrichtung umfassend
- eine Separationseinheit dazu eingerichtet, das gelöste Gas (GG) in eine Gasphase zu überführen um ein Analysegasgemisch (AG) zu erhalten;
- einen Analysesensor (AS) dazu eingerichtet, ein Sensorsignal als Ausgangssignal abhängig von einer Zusammensetzung des Analysegasgemisches (AG) zu erzeugen, wobei die Zusammensetzung des Analysegasgemisches (AG) einer Konzentration eines in eine Gasphase überführten Gases in dem Analysegasgemisch (AG) entspricht;
- eine Pumpvorrichtung (P1, P2) dazu eingerichtet, das Analysegasgemisch (AG) von der Separationseinheit zu dem Analysesensor (AS) zu transportieren;
- eine Auswerteeinheit (AE), welche dazu eingerichtet ist
- eine erste Zwischengröße anhand eines ersten Regressionsmodells, basierend auf dem Sensorsignal, welches von der Konzentration des in die Gasphase überführten Gases in dem Analysegasgemisch (AG) abhängig ist und wenigstens einer Störgröße und einer weiteren Störgröße, zu bestimmen, wobei die erste Zwischengröße eine von der Störgröße und der weiteren Störgröße beeinflusste Konzentration des in die Gasphase überführten Gases ist und die wenigstens eine Störgröße eine Temperatur und ein Feuchtegehalt einer Umgebung des Analysesensors (AS) und die weitere Störgröße eine Strömungsgeschwindigkeit des Isoliermediums (IM) umfasst;
- eine zweite Zwischengröße anhand eines zweiten Regressionsmodells basierend auf der ersten Zwischengröße und wenigstens einer spezifischen Größe der Analysevorrichtung zu bestimmen, wobei die wenigstens eine spezifische Größe der Analysevorrichtung eine Eigenschaft des Analysesensors (AS) oder der Analysevorrichtung umfasst; und
- einen Wert für eine Konzentration des gelösten Gases (GG) in dem Isoliermedium (IM) basierend auf der zweiten Zwischengröße zu bestimmen.

10. Analysevorrichtung nach Anspruch 9, wobei die Separationseinheit eine semipermeable Membran (M) umfasst, durch welche das gelöste Gas (GG) von dem Hochspannungsgerät (HG) in die Analysevorrichtung gelangen kann.

11. Analysevorrichtung nach einem der Ansprüche 9 oder 10, wobei die Auswerteeinheit (AE)
- eine Prozessoreinheit und eine Messeinrichtung zur Bestimmung einer Temperatur der Prozessoreinheit umfasst; und
- dazu eingerichtet ist, eine Temperatur in einer Umgebung des Analysesensors (AS) basierend auf der Temperatur der Prozessoreinheit zu bestimmen.

## Claims

1. Method for analysing a gas (GG) dissolved in an insulating medium (IM) of a high-voltage device (HG), wherein the method comprises
- transferring the dissolved gas (GG) by means of an analysis device into a gas phase to obtain an analysis gas mixture (AG);
- transporting the analysis gas mixture (AG) to an analysis sensor (AS) of the analysis device;
- generating a sensor signal as an output signal of the analysis sensor (AS) depending on a composition of the analysis gas mixture (AG), wherein the composition of the analysis gas mixture (AG) corresponds to a concentration of a gas transferred into a gas phase in the analysis gas mixture (AG);
- determining a first intermediate value based on the sensor signal, which depends on the concentration of the gas transferred into the gas phase in the analysis gas mixture (AG), and at least one disturbance value and a further disturbance value, with the aid of a first regression model, wherein the first intermediate value is a concentration of the gas transferred into the gas phase, the concentration being influenced by the disturbance value and the further disturbance value, and the at least one disturbance value comprises a temperature and a moisture content of an environment of the analysis sensor (AS) and the further disturbance value comprises a flow velocity of the insulating medium (IM) ;
- determining a second intermediate value based on the first intermediate value and at least one specific value of the analysis device with the aid of a second regression model, wherein the at least one specific value of the analysis device comprises a property of the analysis sensor (AS) or the analysis device; and
- determining a value for a concentration of the dissolved gas (GG) in the insulating medium (IM) based on the second intermediate value.

2. Method according to Claim 1, wherein the at least one specific value of the analysis device influences the transfer of the dissolved gas (GG) into the gas phase or a behaviour of the analysis sensor (AS).

3. Method according to any of Claims 1 or 2, wherein the transfer of the dissolved gas (GG) into the gas phase is performed by means of a semipermeable membrane (M) of the analysis device and the at least one specific value of the analysis device comprises a property of the membrane (M), in particular a porosity of the membrane (M) .

4. Method according to any of Claims 1 to 3, wherein the property of the analysis sensor (AS) is a sensitivity, a response time or reaction time of the analysis sensor (AS).

5. Method according to any of Claims 1 to 4, wherein the at least one disturbance value influences a behaviour of the analysis sensor (AS).

6. Method according to any of Claims 1 to 5, wherein determining the second intermediate value is performed additionally based on a specific value of the insulating medium (IM).

7. Method according to Claim 6, wherein the specific value of the insulating medium (IM) influences the transfer of the dissolved gas (GG) into the gas phase.

8. Method according to any of Claims 6 or 7, wherein the specific value of the insulating medium (IM) comprises a solubility of the dissolved gas (GG) in the insulating medium (IM).

9. Analysis device for analysing a gas (GG) dissolved in an insulating medium of a high-voltage device (HG), the analysis device comprising:
- a separation unit configured to transfer the dissolved gas (GG) into a gas phase to obtain an analysis gas mixture (AG);
- an analysis sensor (AS) configured to generate a sensor signal as output signal depending on a composition of the analysis gas mixture (AG), wherein the composition of the analysis gas mixture (AG) corresponds to a concentration of a gas transferred into a gas phase in the analysis gas mixture (AG);
- a pump device (P1, P2) configured to transport the analysis gas mixture (AG) from the separation unit to the analysis sensor (AS);
- an evaluation unit (AE), which is configured
- to determine a first intermediate value with the aid of a first regression model, based on the sensor signal, which depends on the concentration of the gas transferred into the gas phase in the analysis gas mixture (AG), and at least one disturbance value and a further disturbance value, wherein the first intermediate value is a concentration of the gas transferred into the gas phase, the concentration being influenced by the disturbance value and the further disturbance value, and the at least one disturbance value comprises a temperature and a moisture content of an environment of the analysis sensor (AS) and the further disturbance value comprises a flow velocity of the insulating medium (IM);
- to determine a second intermediate value with the aid of a second regression model, based on the first intermediate value and at least one specific value of the analysis device, wherein the at least one specific value of the analysis device comprises a property of the analysis sensor (AS) or the analysis device; and
- to determine a value for a concentration of the dissolved gas (GG) in the insulating medium (IM) based on the second intermediate value.

10. Analysis device according to Claim 9, wherein the separation unit comprises a semipermeable membrane (M), through which the dissolved gas (GG) can pass from the high-voltage device (HG) into the analysis device.

11. Analysis device according to any of Claims 9 or 10, wherein the evaluation unit (AE)
- comprises a processor unit and a measuring device for determining a temperature of the processor unit; and
- is configured to determine a temperature in an environment of the analysis sensor (AS) based on the temperature of the processor unit.

## Revendications

1. Procédé d'analyse d'un gaz dissous (GG) dans un milieu isolant (IM) d'un appareil à haute tension (HG), le procédé consistant à
- transformer le gaz dissous (GG) en une phase gazeuse au moyen d'un dispositif d'analyse afin d'obtenir un mélange de gaz d'analyse (AG) ;
- acheminer le mélange de gaz d'analyse (AG) vers un capteur d'analyse (AS) du dispositif d'analyse ;
- générer un signal de capteur en tant que signal de sortie du capteur d'analyse (AS) en fonction d'une composition du mélange de gaz d'analyse (AG), la composition du mélange de gaz d'analyse (AG) correspondant à une concentration d'un gaz transformé en une phase gazeuse dans le mélange de gaz d'analyse (AG) ;
- déterminer une première grandeur intermédiaire sur la base du signal de capteur, qui dépend de la concentration du gaz transformé en la phase gazeuse dans le mélange de gaz d'analyse (AG) et d'au moins une grandeur perturbatrice et d'une autre grandeur perturbatrice, à l'aide d'un premier modèle de régression, la première grandeur intermédiaire étant une concentration du gaz transformé en la phase gazeuse, influencée par la grandeur perturbatrice et l'autre grandeur perturbatrice, et l'au moins une grandeur perturbatrice comprenant une température et une teneur en humidité d'un environnement du capteur d'analyse (AS) et l'autre grandeur perturbatrice comprenant une vitesse d'écoulement du milieu isolant (IM) ;
- déterminer une seconde grandeur intermédiaire sur la base de la première grandeur intermédiaire et d'au moins une grandeur spécifique du dispositif d'analyse à l'aide d'un second modèle de régression, l'au moins une grandeur spécifique du dispositif d'analyse comprenant une propriété du capteur d'analyse (AS) ou du dispositif d'analyse ; et
- déterminer une valeur d'une concentration du gaz dissous (GG) dans le milieu isolant (IM) sur la base de la seconde grandeur intermédiaire.

2. Procédé selon la revendication 1, dans lequel l'au moins une grandeur spécifique du dispositif d'analyse influence la transformation du gaz dissous (GG) en la phase gazeuse ou un comportement du capteur d'analyse (AS) .

3. Procédé selon l'une quelconque des revendications 1 ou 2, dans lequel la transformation du gaz dissous (GG) en la phase gazeuse est réalisée au moyen d'une membrane semi-perméable (M) du dispositif d'analyse et l'au moins une grandeur spécifique du dispositif d'analyse comprend une propriété de la membrane (M), notamment une porosité de la membrane (M).

4. Procédé selon l'une quelconque des revendications 1 à 3, dans lequel la propriété du capteur d'analyse (AS) est une sensibilité, un temps de réponse ou un temps de réaction du capteur d'analyse (AS).

5. Procédé selon l'une quelconque des revendications 1 à 4, dans lequel l'au moins une grandeur perturbatrice influence un comportement du capteur d'analyse (AS).

6. Procédé selon l'une quelconque des revendications 1 à 5, dans lequel la détermination de la seconde grandeur intermédiaire est en outre effectuée sur la base d'une grandeur spécifique du milieu isolant (IM).

7. Procédé selon la revendication 6, dans lequel la grandeur spécifique du milieu isolant (IM) influence la transformation du gaz dissous (GG) en la phase gazeuse.

8. Procédé selon l'une quelconque des revendications 6 ou 7, dans lequel la grandeur spécifique du milieu isolant (IM) comprend une solubilité du gaz dissous (GG) dans le milieu isolant (IM).

9. Dispositif d'analyse permettant d'analyser un gaz dissous (GG) dans un milieu isolant d'un appareil à haute tension (HG), le dispositif d'analyse comprenant
- une unité de séparation conçue pour transformer le gaz dissous (GG) en une phase gazeuse afin d'obtenir un mélange de gaz d'analyse (AG) ;
- un capteur d'analyse (AS) conçu pour générer un signal de capteur en tant que signal de sortie en fonction d'une composition du mélange de gaz d'analyse (AG), la composition du mélange de gaz d'analyse (AG) correspondant à une concentration d'un gaz transformé en une phase gazeuse dans le mélange de gaz d'analyse (AG) ;
- un dispositif de pompage (P1, P2) conçu pour acheminer le mélange de gaz d'analyse (AG) de l'unité de séparation vers le capteur d'analyse (AS) ;
- une unité d'évaluation (AE), qui est conçue pour
- déterminer une première grandeur intermédiaire à l'aide d'un premier modèle de régression, sur la base du signal de capteur, qui dépend de la concentration du gaz transformé en la phase gazeuse dans le mélange de gaz d'analyse (AG) et d'au moins une grandeur perturbatrice et d'une autre grandeur perturbatrice, la première grandeur intermédiaire étant une concentration du gaz transformé en la phase gazeuse, influencée par la grandeur perturbatrice et l'autre grandeur perturbatrice, et l'au moins une grandeur perturbatrice comprenant une température et une teneur en humidité d'un environnement du capteur d'analyse (AS) et l'autre grandeur perturbatrice comprenant une vitesse d'écoulement du milieu isolant (IM) ;
- déterminer une seconde grandeur intermédiaire à l'aide d'un second modèle de régression sur la base de la première grandeur intermédiaire et d'au moins une grandeur spécifique du dispositif d'analyse, l'au moins une grandeur spécifique du dispositif d'analyse comprenant une propriété du capteur d'analyse (AS) ou du dispositif d'analyse ; et
- déterminer une valeur d'une concentration du gaz dissous (GG) dans le milieu isolant (IM) sur la base de la seconde grandeur intermédiaire.

10. Dispositif d'analyse selon la revendication 9, dans lequel l'unité de séparation comprend une membrane semi-perméable (M) à travers laquelle le gaz dissous (GG) peut passer de l'appareil à haute tension (HG) au dispositif d'analyse.

11. Dispositif d'analyse selon l'une des revendications 9 ou 10, dans lequel l'unité d'évaluation (AE)
- comprend une unité de traitement et un dispositif de mesure pour déterminer une température de l'unité de traitement ; et
- est conçue pour déterminer une température dans un environnement du capteur d'analyse (AS) sur la base de la température de l'unité de traitement.
